# EUROPEAN PATENT APPLICATION

(11) **EP 2 366 431 A1**
(43) Date of publication of application: **21.09.2011**
(21) Application number: 10002971.9
(22) Date of filing: 19.03.2010
(51) Int. Cl.: A61P 9/06, A61K 31/7088, C12N 9/08, C12N 15/113, A61K 31/00

(54) **Myeloperoxidase as a target in atrial fibrillation**

(71) Applicant: Universitätsklinikum Hamburg-Eppendorf, 20246 Hamburg (DE)
(72) Inventor: The designation of the inventor has not yet been filed
(74) Representative: UEXKÜLL & STOLBERG

(57) **Abstract**

The present invention relates to the field of cardiovascular diseases, in particular to diagnosis and treatment of atrial fibrillation. The invention provides inhibitors of expression and/or activity of myeloperoxidase for treatment of atrial fibrillation as well as kits and methods for diagnosing susceptibility to atrial fibrillation in a subject and for identifying subjects amenable to treatment of atrial fibrillation with an inhibitor of myeloperoxidase. The invention further relates to a method for identifying a compound for treatment of atrial fibrillation, and use of such an inhibitor for the preparation of a medicament for treatment of atrial fibrillation.

## Description

The present invention relates to the field of cardiovascular diseases, in particular to diagnosis and treatment of atrial fibrillation. The invention provides inhibitors of expression and/or activity of myeloperoxidase for treatment of atrial fibrillation as well as kits and methods for diagnosing susceptibility to atrial fibrillation in a subject and for identifying subjects amenable to treatment of atrial fibrillation with an inhibitor of myeloperoxidase. The invention further relates to a method for identifying a compound for treatment of atrial fibrillation, and use of such an inhibitor for the preparation of a medicament for treatment of atrial fibrillation.

Observational clinical and *ex vivo* studies have established a strong association between atrial fibrillation (AF) and inflammation¹. However, whether inflammation is cause or consequence of AF and which specific inflammatory mediators may increase the atria's susceptibility to fibrillate remained elusive.

Atrial fibrillation (AF) remains the most common arrhythmia in humans and stands out as a major contributor to morbidity and mortality²⁻⁴. Despite recent advances in pharmacological and invasive strategies to treat AF, the underlying pathophysiology remains incompletely understood. Past attempts to elucidate the pathophysiology of AF mainly focused on the electrical substrate, with an increasing number of studies now suggesting that AF shares pathophysiological characteristics of an inflammatory disease. Clinical investigations report multiple associations between the vulnerability for AF and circulating levels of cytokines, C-reactive protein (CRP), complement ⁵⁻⁷ and - in particular - the activation state of leukocytes⁸⁻¹⁰. Whether this inflammatory phenotype represents an epiphenomenon in AF or is causally linked to the initiation and progression of AF had so far remained undefined.

Herein, we provide experimental and clinical evidence for mechanistic involvement of myeloperoxidase (MPO), a heme enzyme abundantly expressed by neutrophils, in the pathophysiology of AF: MPO knock-out mice pretreated with angiotensin II (AngII) to provoke leukocyte activation, revealed attenuated atrial tissue levels of the MPO product 3-chlorotyrosine, reduced activity of matrix metalloproteinases, and blunted atrial fibrosis as compared to wild type mice. Upon right atrial electrophysiological stimulation, MPO knock-out mice were protected from atrial fibrillation, which was reversed when MPO was restituted. Humans with AF exhibited higher plasma levels of MPO and revealed an increased MPO burden in right atrial tissue as compared to individuals devoid of AF. Here, MPO co-localized with markedly increased formation of 3-chlorotyrosine. In conclusion, the current findings demonstrate that MPO is a critical prerequisite for structural myocardial remodeling and atrial fibrosis, which predisposes to increased vulnerability for atrial fibrillation.

The present invention thus addresses the need for providing a new target in atrial fibrillation, which is in particular useful for providing a medicament for treatment of atrial fibrillation.

The invention provides an inhibitor of expression and/or activity and/or atrial deposition of myeloperoxidase for use in the treatment of atrial fibrillation of a subject in need thereof. The invention also relates to a method of treating atrial fibrillation of a subject in need thereof, comprising administering an inhibitor of expression and/or activity and/or atrial deposition of myeloperoxidase to the subject in an effective amount.

Myeloperoxidase (MPO) (EC 1.11.1.7) is a heme protein synthesized during myeloid differentiation that constitutes the major component of neutrophil azurophilic granules. Produced as a single chain precursor, myeloperoxidase is subsequently cleaved into a light and heavy chain. The mature myeloperoxidase is a tetramer composed of 2 light chains and 2 heavy chains. This enzyme produces hypohalous acids central to the microbicidal activity of netrophils. MPO generates reactive oxygen species as part of its function in innate host defense mechanisms, however, in an unregulated state, it is capable of causing tissue destruction, inflammation, and has previously been implicated in numerous diseases, including atherosclerosis, cancer, and Alzheimer's disease. The sequence of human myeloperoxidase is disclosed, e.g., in Morishita et al. (1987 JBC, 262, 15208-15213) or Yamada et al. (1990, Biochem Biophys Res Commun.166(2):852-9). In the context of this invention, MPO relates to MPO derived from human or animal (e.g, mouse, rat, rabbit, sheep, goat, swine, dog, horse, cattle, guinea pig, ape). Preferably, MPO has at least 80% or at least 90% sequence identity to the sequence of human myeloperoxidase. Preferably, MPO is human MPO.

The inhibitor may be an inhibitor of expression of myeloperoxidase selected from the group comprising siRNA and antisense RNA specifically inhibiting expression of myeloperoxidase. Methods for generating siRNA and/or antisense RNA are known in the state of the art.

Inhibitors of activity of myeloperoxidase are known in the state of the art. In the context of the invention, an inhibitor can be an inhibitor of myeloperoxidase described by, e.g., Kettle et al. 1995, Malle et al. 2007, Jantschko et al., 2005, Pincemail et al., 1988 or US 2008096929.

In one embodiment, the inhibitor of activity of myeloperoxidase is selected from the group comprising quercitin, methimazole, rutin, rutin sulfate, troxerutin, a hydroxamic acid (e.g., SHA, BHA), a bencoic acid hydrazide or hydrazine (e.g., 4-aminobenzoic acid hydrazide, isonicotinic acid hydrazide (isoniazid)), an indole or tryptamine derivative (e.g., 5-fluorotryptamine, 5-chlorotryptamine), flufenamic acid, diclofenac, niflumic acid, tenoxicam, piroxicam, melatonin, a derivative of 2,4-dihydro-[1,2,4]triazole-3-5 thione, a serum inhibitor of myeloperoxidase, as, e.g., disclosed by Yea et al., 1991, and an antagonistic antibody to myeloperoxidase. An antagonistic antibody to myeloperoxidase can be a mouse, human, humanized or chimeric antibody, in particular, a monoclonal antibody. It may be recombinantly expressed and/or linked to additional moieties, e.g., pegylated. For example, it may be produced as a fusion protein with a tag, such as a His-Tag. Antibodies comprise antibody fragments, e.g., Fab, F(ab)2, or scFv antibodies. An inhibitor, such as an antagonistic antibody can be identified in an assay of MPO activity such as described below or disclosed in Malle et al. 2007.

US 2008096929 discloses derivatives of 2,4-dihydro-[1,2,4]triazole-3-5 thione or pharmaceutically acceptable salts thereof, which may be used in the context of the present invention.

The inhibitor may also inhibit and/or reverse atrial deposition of myeloperoxidase. The inventors have shown that heparin may contribute to dissociation of MPO from deposits thereof, and can thus be used as an inhibitor in he context of the invention.

The inhibitor may also inhibit binding of MPO to the MPO binding site of apolipoprotein A-I (apoA-1), wherein the MPO binding site is located in the helix 8 domain of apoA1, and wherein the agent is chosen from an isolated apoA-1 peptide fragment, a modified form of the apo-1 fragment comprising one or more D amino acids, a retro-inverso form of the apoA-1 peptide fragment, an organo-mimetic of the apoA-1 peptide fragment, a peptide-mimetic of the apoA1 peptide fragment, or a nucleic acid encoding the apo A-I peptide fragment, as taught in WO2006020498.

An inhibitor may be a protein or peptide or a non-peptide small chemical compound.

Of course, one or more inhibitor may be used in combination, i.e., administered at the same time point or before or after administration of the other agent. Preferably, administration of two or more agents is within 1 hour, 2 hours, 4 hours, 8 hours, 12 hours, 24 hours, 2 days, 3, days, 4 days or a week. In the context of this invention, "a" is not to be understood to be limited to "one", except where expressly defined. "A" may also refer to two or more, three or more or four or more.

Preferably, the inhibitor is pharmaceutically acceptable, e.g., has acceptable toxicologic characteristics for treatment of a subject, e.g., a human subject. An inhibitor may be targeted to the heart, in particular the atria, e.g., by linking it to an antibody capable of specifically binding to an antigen expressed in this region or by administration (e.g. injection/infusion) to this region, which may reduce side effects. Preferably, the inhibitor is to be administered with a pharmaceutically acceptable carrier and/or excipients.

A subject preferably is a human subject, however, it may be a mouse, rat, rabbit, sheep, goat, swine, dog, horse, cattle, guinea pig, or ape. A subject may be a patient.

In the context of the present invention, the treatment may be prophylactic and/or therapeutic treatment. Treatment thus includes prevention. Preventing includes, e.g. reducing the severity and/or incidence of atrial fibrillation. In one embodiment, the inhibitor is for prevention of atrial fibrillation. The subject may have been diagnosed with lone atrial fibrillation, lone atrial fibrillation or with atrial fibrillation in combination with one, two, three or four diseases selected from coronary artery disease and/or hypertension and/or heart failure and/or valvular disease. Valvular disease may be mitral valve disease. The patient may also have been diagnosed to be susceptible to one or more of these diseases. For example, the patient may have had atrial fibrillation, and the medicament may be for preventing further atrial fibrillation. A large patient group susceptible to atrial fibrillation does not have coronary artery disease and/or hypertension and/or heart failure and/or valvular disease. However, patients having coronary artery disease and/or hypertension and/or heart failure and/or valvular disease may be susceptible to atrial fibrillation, so they may be treated in the context of this invention. In the context of the invention, treatment of atrial fibrillation may or may not comprise treatment of atrial fibrosis. The present invention thus identifies a new patient group for targeting MPO.

The present invention also relates to a method for diagnosing susceptibility to atrial fibrillation in a subject, comprising determining presence and/or activity of myeloperoxidase in a sample obtained from the subject. Susceptibility may be the risk of developing or having atrial fibrillation. In one embodiment, the invention provides a diagnostic test for characterizing a subject's risk of developing or having atrial fibrillation.

In another embodiment, the invention provides a method/test for identifying subjects amenable to treatment with an inhibitor of MPO as defined above, comprising determining presence and/or activity of myeloperoxidase in a sample obtained from the subject.

The test may comprise determining the level of myeloperoxidase (MPO) activity in a bodily sample obtained from the individual or subject. For example, the InnoZyme™ Myeloperoxidase Activity Kit, Merck, is a kit which may be used for quantitative measurements of MPO activity in a sample. The kit may also be used to screen enzyme inhibitors. The InnoZyme Myeloperoxidase assay uses a specific polyclonal antibody immobilized on a 96-well plate to capture MPO.

In another embodiment, the test comprises determining the level of myeloperoxidase (MPO) activity by determining the level of one or more select MPO-generated oxidation products in a sample obtained from the subject. The select MPO-generated oxidation products may be 3-chlorotyrosine, dityrosine, nitrotyrosine, methionine sulphoxide or MPO-generated lipid peroxidation products.

In another embodiment, the presence (and level) of MPO may be determined by ELISA or Western blot, e.g., as described below. The test may comprise determining the level of MPO mass in a sample obtained from the subject.

Levels of MPO activity, MPO presence or mass, or the select MPO-generated oxidation product in samples from the subject are then compared to a predetermined value that is derived from measurements of MPO activity, MPO presence or mass, or the select MPO-generated oxidation product in comparable samples obtained from the general population or a select population of human subjects. Such comparison characterizes the subject's risk of developing atrial fibrillation and/or atrial fibrosis. A higher than average level indicates that the subject is susceptible to atrial fibrillation, and that the subject is amenable to treatment of atrial fibrillation with an MPO inhibitor.

Tests taught in US 2008009020 may be used in the context of this invention.

The sample may be a blood sample, a plasma sample, a biopsy from the myocard and a specimen derived from right or left atrial appendages.

In one embodiment, it is determined that a subject is amenable to treatment with an inhibitor of myeloperoxidase by determining presence and/or activity of myeloperoxidase in a sample obtained from the subject, e.g., as described above, and amenable subjects are then to be treated with a myeloperoxidase inhibitor as described above.

Also disclosed is a method for identifying a compound for treatment of atrial fibrillation, comprising detecting activity or expression of myeloperoxidase in the presence and absence of a candidate substance under conditions suitable for myeloperoxidase activity, wherein the candidate substance is suitable for treatment of atrial fibrillation if it inhibits activity or expression of the myeloperoxidase. Also disclosed is a medicament or compound for treatment of atrial fibrillation obtainable by the method for identifying a compound for treatment of atrial fibrillation.

Also disclosed is a method of preparing a medicament for treatment of atrial fibrillation, comprising carrying above and formulating a compound identified as suitable for treatment of atrial fibrillation with a pharmacologically acceptable carrier and/or excipient.

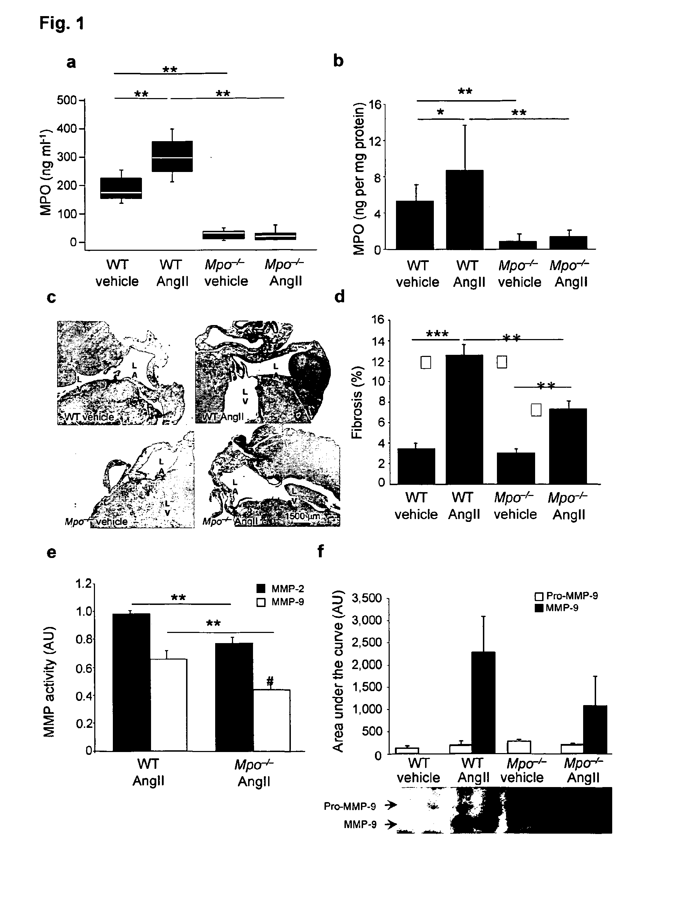

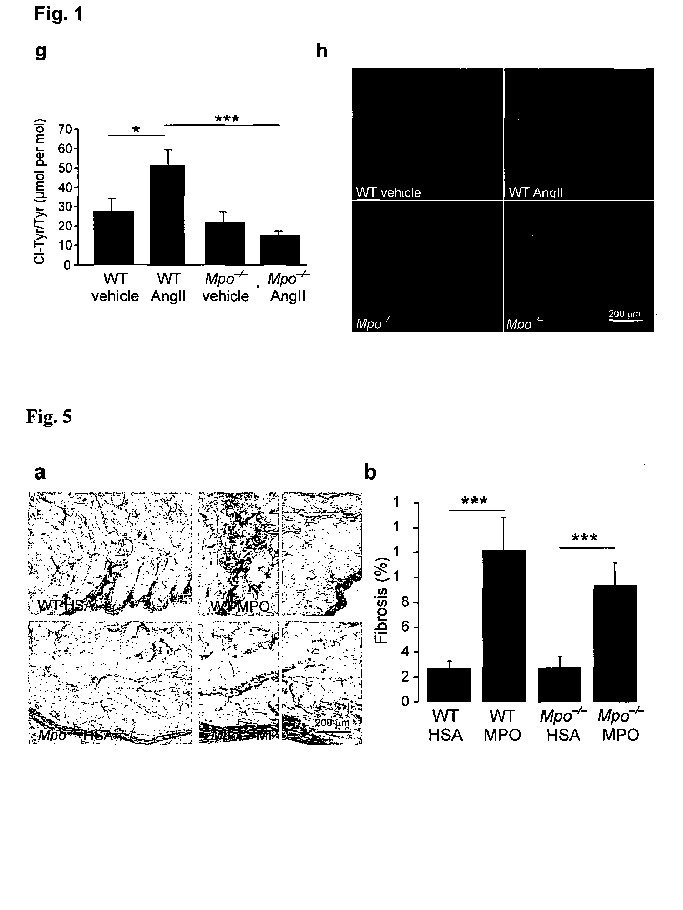

In the context of the invention, to elucidate the role of MPO for structural remodeling of the atria, histological and liquid-chromatography-mass-spectrometry (LC-MS)-based studies of the atria from MPO-deficient mice *(Mpo*^{*-*/}*⁻)* and C57BL/6 wild-type (WT) mice were performed. In WT mice, a 2 week subcutaneous infusion of angiotensin II (AngII), an effector of atrial remodeling and fibrosis¹⁵⁻¹⁸, substantially increased circulating MPO plasma levels and elevated myocardial deposition of MPO **(****Fig. 1a**, **b****)**. Picrosirius red staining revealed marked attenuation of atrial fibrosis in AngII treated *Mpo* ^{*-*/*-*} mice compared to AngII treated WT mice **(****Fig. 1c**, **d****)**. In support of a MPO-dependent increase in atrial fibrosis, *Mpo*^{*-*/*-*} mice, which were intravenously challenged with recombinant MPO via osmotic pumps developed a similar degree of atrial fibrosis, which thus developed independent of AngII supplemenation **(****Fig. 5a****, b).** To further characterize the molecular basis of MPO-dependent atrial remodeling, atrial MMP-2 and MMP-9 activity was determined, both of which were profoundly reduced in AngII treated *Mpo^{-l-}* mice as evidenced by zymography and immunoblotting for MMP-9, respectively **(****Fig. 1e**, **f****)**. RT-PCR excluded differences in MMP-9 mRNA expression between the four groups (data not shown). Since MMPs are activated by HOCl, atrial tissue levels of 3-chlorotyrosine (Cl-Tyr) were determined, a specific footprint of MPO-dependent HOCl formation. Mass spectrometry-based quantitative analysis and immunohistochemical studies both demonstrated markedly reduced formation of Cl-Tyr in *Mpo*^{*-*/*-*} mice following AngII treatment **(****Fig. 1g**, **h****)**, thus indicating lower HOCl bioavailability in these animals.

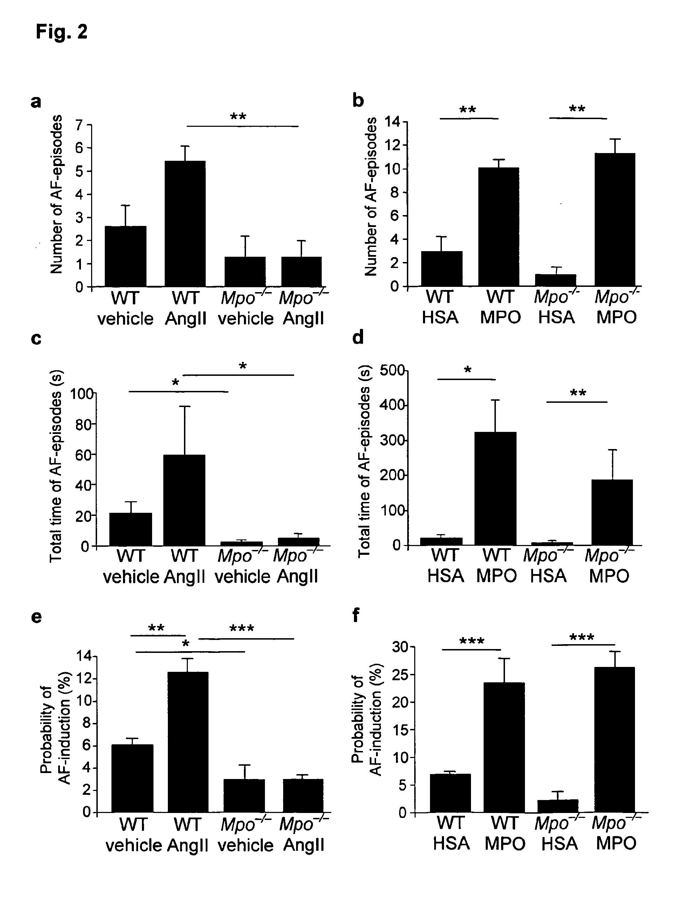

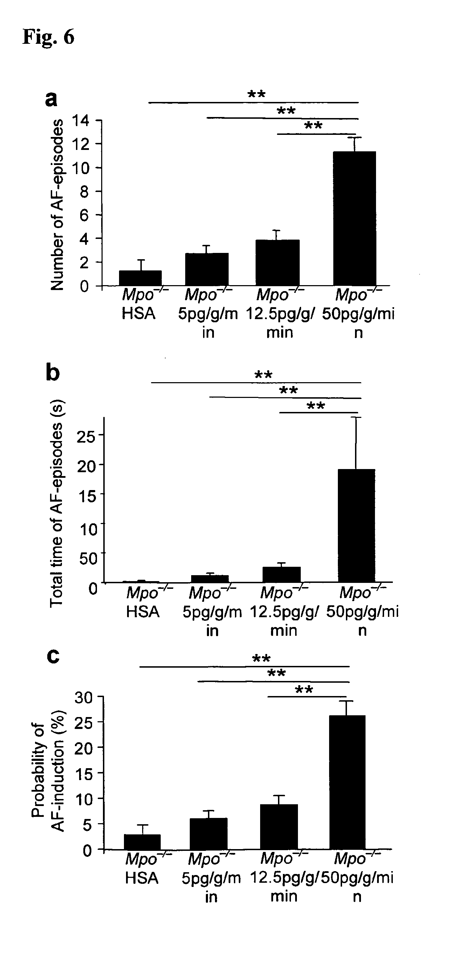

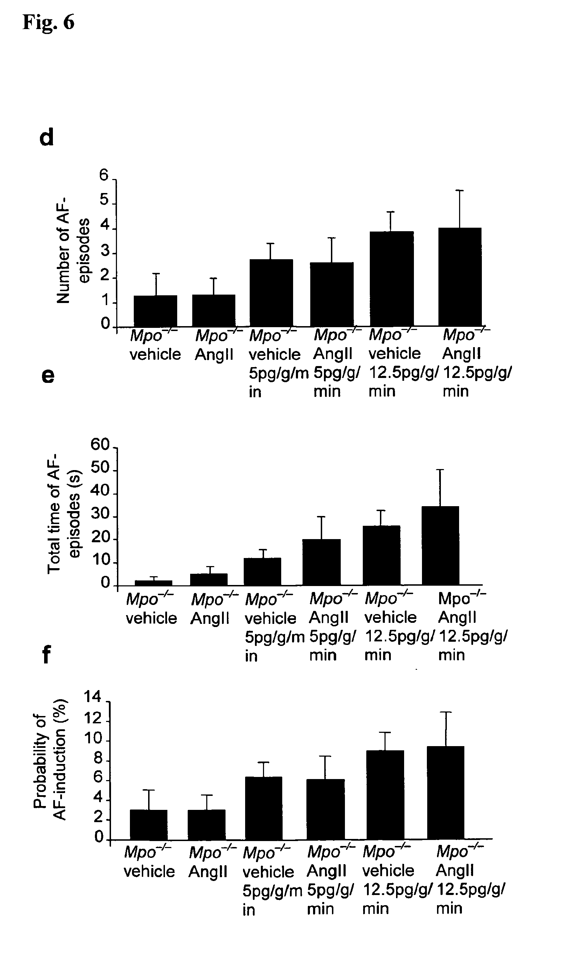

To test whether MPO-dependent structural remodeling also translated into increased electrical instability and thus higher vulnerability for the development of AF, *Mpo*^{*-*/*-*} and WT mice were subjected to electrophysiological investigation *in vivo*¹⁹. Upon right atrial electrophysiological stimulation WT mice challenged with AngII revealed a higher susceptibility for AF, with an increased number and duration of AF-episodes as well as an increased probability of AF induction. In sharp contrast, AngII treatment did not affect the inducibility of AF in *Mpo*^{*-*/*-*} mice, reflecting almost complete protection from AF in *Mpo*^{*-*/*-*} mice. Continuous intravenous supplementation of recombinant MPO via minipumps however reestablished the vulnerability for atrial fibrillation in *Mpo*^{*-*/*-*} mice **(****Fig. 2a****-f),** which demonstrated to be dose dependent and irrespective of additional delivery of AngII **(****Fig. 6****).** This infers that MPO by itself, independent of the leukocyte and of AngII promotes electrical instability in the atria.

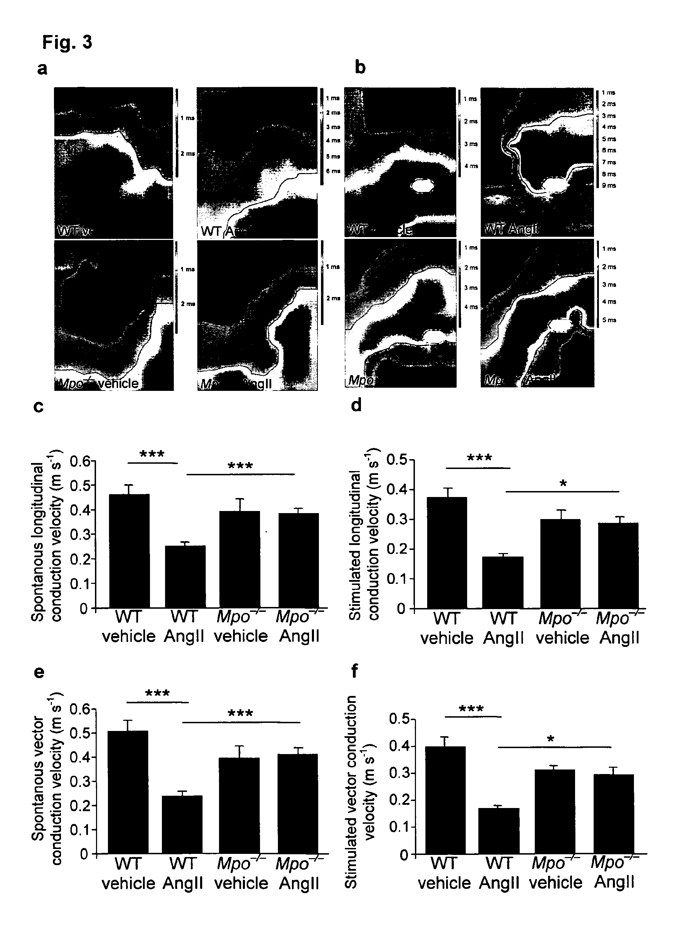

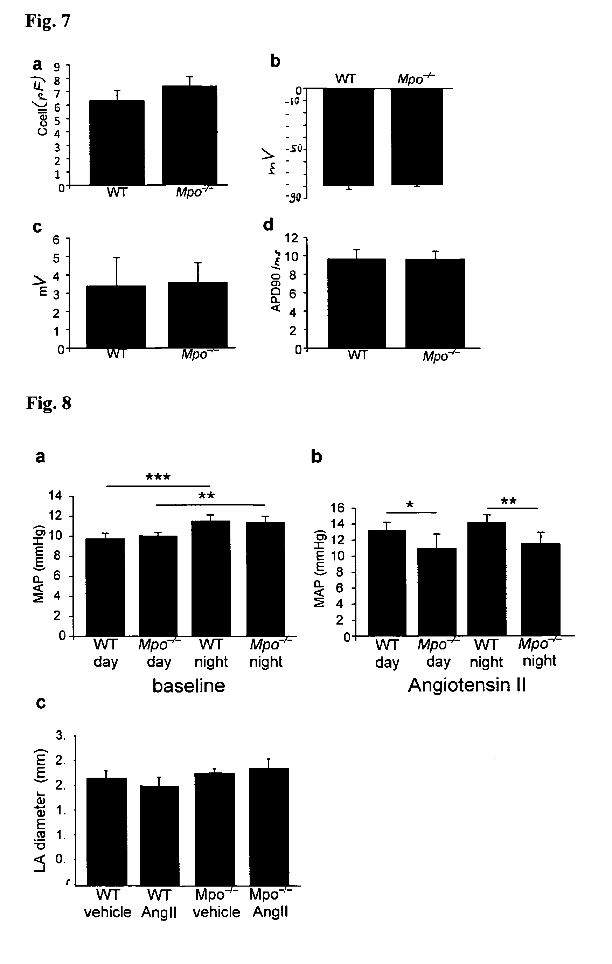

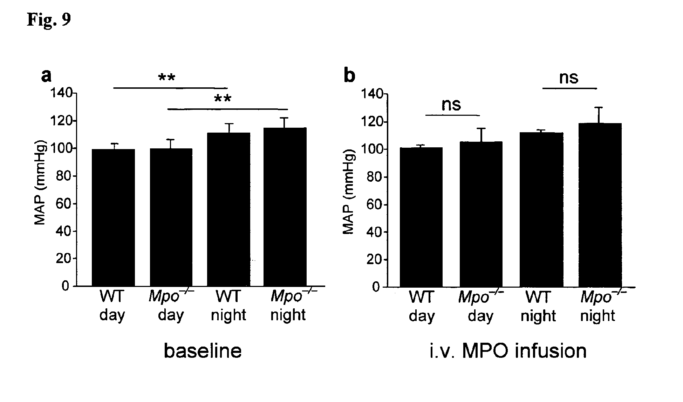

To further evaluate whether MPO-dependent atrial fibrosis increased atrial conduction inhomogeneity, an electrophysiological hallmark of AF²⁰, epicardial activation mapping was performed in Langendorff perfused hearts from WT and *Mpo*^{*-*/*-*} mice²¹ . Atria from AngII treated Mpo^{*-*/*-*} mice - in sharp contrast to AngII challenged WT mice - showed no retardation in conduction velocity **(****Fig. 3a****-f),** which complemented the histological observations of blunted atrial fibrosis in MPO naïve animals. Accordingly*, in vivo* surface ECG tracings showed a significant prolongation of P wave duration in WT mice upon AngII treatment (p<0.001, **Table 1).** Studies on isolated myocytes from WT and *Mpo*^{*-*/*-*} mice excluded differences in the cells' electrical homogeneity **(****Fig. 7a****-d);** also, serum potassium levels between the different treatment groups remained unaffected (data not shown). Given the well documented effects of AngII on blood pressure, the cytokine-like, leukocyte activating properties of AngII²² and the established NO oxidizing properties of MPO^{23,24}, *in vivo* radiotelemetric blood pressure measurements and echocardiographic assessment of left atrial dimensions were performed in mice. Whereas baseline blood pressure was not different between groups **(****Fig. 8a****),** the blood pressure elevating effect of AngII in WT mice was blunted in *Mpo*^{*-*/*-*} mice **(****Fig. 8b****).** Conversely, blood pressure in *Mpo*^{*-*/*-*} animals reconstituted with MPO in the absence of AngII remained unaltered as compared to vehicle treated animals **(****Fig. 9a****, b),** inferring that MPO-dependent modulation of vascular tone on the one hand and structural atrial remodeling on the other is differentially regulated. Echocardiographic studies revealed no changes in atrial diameters across groups **(****Fig. 8c****).** Taken together, these observations reinforce that electrical instability evoked by MPO does not simply reflect changes in systemic vasomotion and atrial dimensions, respectively, but is functionally linked to the structural transformation within atrial tissue.

To evaluate the MPO-dependent effects on atrial remodeling in humans, individuals with pacemakers with and devoid of AF (*n* = 42) were followed for a period of 114±73 days **(Table 2).** The inclusion of individuals with a new generation dual-chamber pacemaker allowed for continuous atrial sensing over the entire follow-up period to detect atrial fibrillation. Twenty-four individuals displayed AF (AF-burden: 45.2 ± 39.9%, equaling 871 ± 1,541 hours of AF). Individuals with AF revealed significantly higher MPO plasma levels (503.1 [IR: 404.6-880.7] vs. 437.8 [IR: 348.9-488.0 pmol 1⁻¹; *P* = 0.025), which correlated with the AF burden (Spearman's rho: 0.5; *P* = 0.002). Circulating levels of elastase, an enzyme stored in the same granules of neutrophils as MPO, were also significantly elevated in individuals with AF (50.5±23.9 vs. 38.3±12.4 ng ml⁻¹; *P* = 0.039) again correlating with the AF burden (r: 0.5; *P* = 0.004), further underscoring that increased MPO levels are a reflection of leukocyte activation and degranulation.

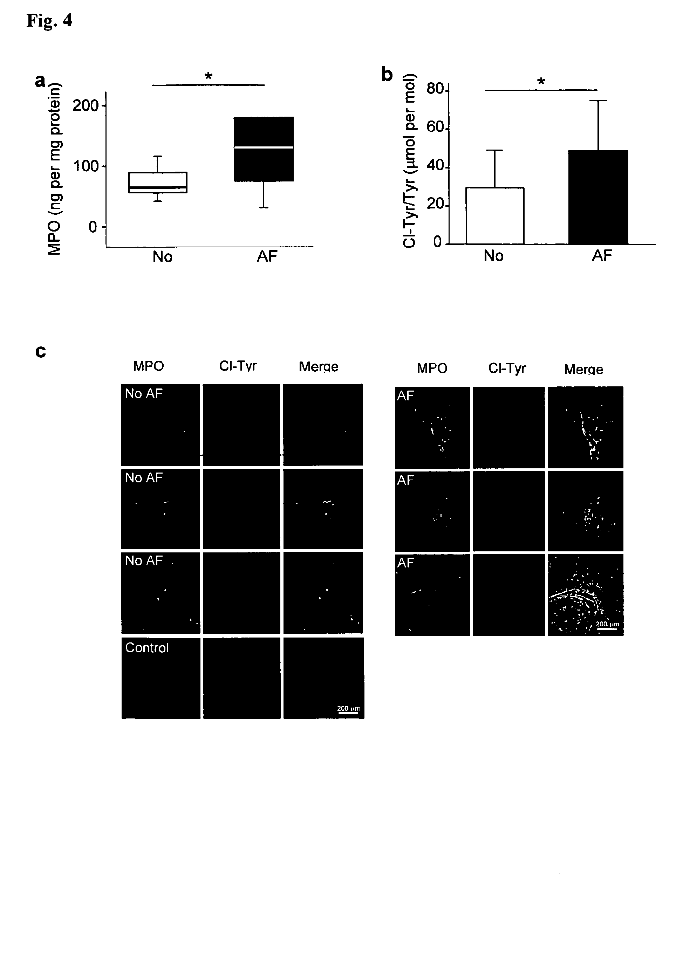

To test local sequestration of MPO within the atria, MPO concentration was measured and immunohistochemical studies were performed using specimens derived from right atrial appendages of a cohort of individuals undergoing coronary bypass surgery (*n* = 27, **Table 3).** Individuals with concomitant AF exhibited substantially increased atrial MPO deposition **(****Fig. 4a****)** and enhanced tissue 3-chlorotyrosine content **(****Fig. 4b****),** which co-localized with MPO as evidenced by immunohistochemistry **(****Fig. 4c****),** further underscoring the relationship between MPO and posttranslational protein modification.

The current work suggests that MPO is linked to the pathophysiology of atrial fibrillation in that (i) MPO is found to be increased systemically and locally in humans and animals with AF; (ii) MPO leads to structural changes, namely atrial fibrosis, known to be associated with the development of AF; (iii) genetic depletion of MPO proved to profoundly decrease vulnerability for atrial fibrillation, whereas (iiii) reconstitution of MPO reestablished the vulnerability for atrial fibrillation in an Ang II independent fashion. The present work thus for the first time shows a causal effect of MPO in this disease and opens up therapeutic possibilities.

Atrial fibrosis is considered a key prerequisite for the initiation and propagation of atrial fibrillation ²⁵⁻²⁷ and serves as a substrate for AF²⁰. In search of subcellular mediators linking structural changes in the atria and the susceptibility for AF, AngII is of central significance. AngII, generated either systemically or locally in the atria, propagates atrial dilatation, fibrosis and ultimately AF¹⁷ . The current data now extend the understanding of the contributory role of AngII in AF and reveal that AngII also elicits pro-fibrotic and pro-arrhythmic properties independent of its direct effects on cardiac myocytes: *Mpo*^{*-*/*-*} mice revealed markedly reduced atrial fibrosis and were almost completely protected from the AngII-dependent increase in atrial vulnerability to fibrillate - both of which was dose dependently reversed upon restitution of MPO.

In search of the molecular mechanisms underlying MPO-dependent atrial fibrosis, atrial MMP activity became of significance. Increased activity of MMPs has been firmly linked to elevated atrial ECM turnover, fibrosis and AF¹¹. MPO regulates MMP activity via posttranslational modification mediated by its specific oxidant HOCl in a concentration-dependent manner^{12,13}. Here we measured 3-chlorotyrosine, a post-translational modification of proteins by the MPO-generated oxidant HOCl²⁸ and show marked increase within atrial tissue of WT mice. The reduction in atrial tissue 3-chlorotyrosine content from *Mpo*^{*-*/*-*} mice was accompanied by substantially reduced endogenous MMP activity in atria from *Mpo*^{*-*/*-*} animals, consistent with MPO-dependent regulation of MMP activity as a critical contributor to increased ECM turnover and atrial fibrosis.

The electrophysiological data obtained from vehicle, AngII and MPO treated *Mpo*^{*-*/*-*} mice now embrace these structural findings and identify a specific inflammatory mediator to be critically linked to AF. These findings support the notion that MPO binding to the atria, protein oxidation and atrial fibrosis is a critical requirement for the development of AF.

Importantly, the findings obtained from animals were corroborated in human pathology, which showed elevated MPO plasma levels in individuals with AF and increased atrial deposition of MPO and the posttranslational oxidation product 3-chlorotyrosine, both of which co-localized.

Certainly, other MPO-catalyzed oxidation reactions linked to increased myocyte apoptosis or inactivation of redox-sensitive enzyme systems such as myofibrillar creatine kinase²⁹ may also have favored AF-protection in MPO naïve animals. Furthermore, MPO-dependent oxidants have been linked to inhibition of inhibitors of protease cascades such as alpha 1 antitrypsin³⁰, and tissue inhibitors of matrix metalloproteinases³¹. Also, elevated arterial blood pressure in AngII treated, MPO-competent animals per se may have further nurtured atrial fibrosis, even if MPO supplementation per se did not affect blood pressure. Taken together, the evidence provided herein revealing atrial accumulation of MPO, accompanied by augmented fibrosis in animal models and humans with AF suggests that MPO is intimately involved in the pathophysiology of AF. Given the for the most part unsatisfactory and mainly ion channel directed pharmacologic strategies to treat AF, MPO has now shown to be not only a critical mediator and diagnostic indicator, but also as a target of treatment in this disease.

### Figure Legends

**Figure 1** *Mpo*^{*-*/*-*} mice exhibited less atrial fibrosis, reduced matrix metalloproteinase activity and lower chlorotyrosine bioavailability following AngII treatment. (**a**) MPO plasma levels in WT mice, as determined by ELISA. *N*= 14-19 **P* < 0.05; ***P* < 0.01. (**b**) AngII led to a significant increase in myocardial MPO deposition. (**c**) Quantification of atrial fibrosis in *Mpo*^{*-*/*-*} and WT mice by picrosirius polarization method. Images are representative of 10 images from 8-10 mice per group. LA: left atrium. LV: left ventricle. (**d**) Quantitative assessment of atrial fibrosis in saline (vehicle) or AngII treated WT and *Mpo*^{*-*/*-*} mice. *N* = 8-12, ***P* < 0.01, ****P* < 0.001. (**e**) MMP-2 and 9 activity in AngII treated WT and *Mpo*^{*-*/*-*} mice. *N*= 7, ***P* < 0.01. (**f**) Immunoblot of pro-MMP-9 and MMP-9 in mouse atrial tissue. (**g**) LC-MS-based quantitative assessment of 3-chlorotyrosine (Cl-Tyr) in atrial tissue in WT and *Mpo*^{*-*/*-*} mice. *N =* 10, **P* < 0.05, ***P* < 0.01. (**h**) Immunostaining of atrial Cl-Tyr in Ang II/saline treated WT and *Mpo*^{*-*/*-*} mice. All data in **b, d, e**, and **g** are means ± standard deviation. Statistical analyses were performed using ANOVA followed by Bonferroni post hoc test. Data in a are presented as median and interquartile range. Kruskal-Wallis followed by Mann-Whitney U post hoc test was used for statistical analysis.
**Figure 2** Analysis of AF inducibility in *Mpo*^{*-*/*-*} and WT mice *in vivo.* Following pretreatment with AngII or saline (vehicle) for 14 days (*n* = 10-16 per group) or MPO or human serum albumin (HSA) for 7 days (n = 6-9 per group), WT and *Mpo*^{*-*/*-*} mice underwent electrophysiological investigation. (**a,b**) Quantification of number of AF-episodes. ***P* < 0.01. (**c,d**) Total time of AF-episodes. **P* < 0.05, ***P* < 0.01. (**e,f**) Probability of induction of AF. Probability of AF induction was defined as inducible episodes divided by total testing maneuvers applied in the groups. **P* < 0.05, ***P* < 0.01, ****P* < 0.001. All data are means ± standard deviation. Statistical analysis was performed using ANOVA followed by Bonferroni post hoc test.
**Figure 3** Epicardial mapping of Langendorff-perfused hearts of AngII or saline (vehicle) treated WT or *Mpo*^{*-*/*-*} mice. (**a**) Representative examples of spontaneous and (**b**) stimulated conduction properties of epicardial activation mapping. White ovals indicate stimulations sites. (**c**) Quantification of spontaneous longitudinal conduction velocity. (**d**) Stimulated longitudinal conduction velocity. (**e**) Spontanous and (**f**) stimulated vector conduction velocity. *N* = 5-13 per group, **P* < 0.05, ****P* < 0.001. All data are means ± standard deviation. Statistical analysis was performed using ANOVA with Bonferroni post hoc test.
**Figure 4** Atrial MPO levels and protein-bound 3-chlorotyrosine in individuals with and devoid of AF. (**a**) Quantitative determination of MPO content in tissue homogenates of right atrial appendages of individuals undergoing on-pump coronary bypass surgery by ELISA normalized to protein concentration (no AF: *n* = 17, AF: *n* = 10). Data presented as median (line) and interquartile range (box), whiskers indicate 5% and 95% percentiles, **P* < 0.05. Statistical analyses were performed using Mann Whitney U test. (**b**) LC-MS-based quantitative assessment of 3-chlorotyrosine (Cl-Tyr) in atrial tissue of the same individuals. Data are means ± standard deviation. **P* < 0.05. Unpaired student's t-test was used for statistical analysis. (**c**) Immunofluorescent staining of human right atrial appendage tissue. Left panel shows three representative examples of MPO and the MPO-specific oxidant Cl-Tyr from different individuals without documented AF. Right panel shows three representative images of different individuals with AF.
**Figure 5**. Assessment of atrial fibrosis in WT and *Mpo-*/*-* mice following i.v. HSA or MPO treatment for 7 days. (a) Representative picrosirius red stained atria. (b) Quantitative analysis. N=6-8, ***P<0.001. ANOVA. All data are expressed as means ± standard deviation.
**Figure 6****.** Analysis of AF inducibility in Mpo-/- mice following 7 days of continuous i.v. MPO treatment in-vivo. MPO dose is given as pg/g body weight/min (n=10-15 per group). (**a**) Quantification of number of AF-episodes. **P<0.001. P for trend: <0.001 (**b**) Total time of AF-episodes. **P<0.001. P for trend: <0.001 (**c**) Probability of induction of AF. **P<0.001. P for trend: <0.001. (d-f) No effect of Ang II on number of AF-episodes (**d**), total time of AF-episodes (e) and probability of AF-induction (**f**) also at lower doses of MPO. All data are means ± standard deviation. ANOVA followed by Bonferroni post hoc test.
**Figure 7**. Electrophysiological investigations in isolated cardiomyocytes. (**a**) Cell capacity (Ccell) in cardiomyocytes isolated from untreated WT and Mpo-/- mice. (**b**) Quantification of resting membrane potential. (**c**) Overshoot in WT and Mpo-/- mice. (**d**) Duration of action potential at 90% repolarization (APD90). All data are expressed as means ± standard deviation. Unpaired Stundent's t-test.
**Figure 8**. (**a**) Continuous radiotelemetric blood pressure measurements in WT and *Mpo-*/*-* mice at baseline and (**b**) following AngII treatment. n=6-7, *P<0.05, **P<0.01, ***P<0.001. ANOVA. (**c**) Echocardiographic assessment of left atrial diameter in WT and *Mpo-l-* mice following saline (vehicle) or AngII treatment. n=8, P=non-significant. ANOVA for intergroup comparisons. Paired student's t-test for day to night difference. All data are expressed as means ± standard deviation.
**Figure 9**. (**a**) Continuous radiotelemetric blood pressure measurements in WT and *Mpo-l-*mice at baseline and (**b**) following continous i.v. MPO treatment for 7 days. N=5-6. **P<0.01. ns P>0.05. ANOVA for intergroup comparisons. Paired student's t-test for day to night difference. All data are expressed as means ± standard deviation.

### Examples

*Angiotensin II and myeloperoxidase treatment of mice.* We treated male C57BL/6J (WT) and MPOtmllus mice *(Mpo*^{*-*/}*⁻)* on a C57BL/6 background, aged 12-15 weeks, via ALZET osmotic pumps with angiotensin II (AngII, 1.5 ng/g body weight/minute) or saline (vehicle) for 14 days prior to electrophysiological investigation (EPI). We treated another cohort of WT and *Mpo*^{*-*/*-*} mice via ALZET osmotic pumps with a connected jugular catheter with recombinant MPO (5.0, 12.5 and 50 pg/g body weight/minute, which we calculated to generate physiological plasma levels of MPO ranging between 100 and 1100 pM, respectively) or recombinant human serum albumin (HSA) for 7 days in combination with either AngII or saline prior to EPI. Animal experiments, conformed with the Guide for the Care and Use of Laboratory Animals published by the US National Institutes of Health and were approved by the review board of Hamburg and Bonn.

*Electrophysiological investigation (EPI).* We examined mice electrophysiologically using a single catheter technique ^{19,32}. We analyzed standard ECG-parameters obtained by a 6-lead-surface-ECG. EPI included transvenous atrial and ventricular recording and stimulation²¹. For induction of AF, we performed atrial and ventricular burst and programmed atrial stimulations. We defined atrial fibrillation as rapid and fragmented atrial electrograms with irregular AV-nodal conduction for >1 s. AF-episodes persisting >1 min were classified as sustained AF. Number, duration, and probability of inducible AF-episodes were analyzed.

*Epicardial mapping of Langendorff-perfused hearts.* We determined spontaneous and stimulated myocardial conduction velocities and characteristics of Langendorff perfused hearts by epicardial activation mapping using a 128-electrodearray²¹.

*Ex vivo electrophysiology studies.* We isolated atrial myocytes enzymatically from WT and *Mpo*^{*-*/*-*} mice and recorded action potentials using the ruptured patch whole-cell patch-clamp technique³³.

*Assessment of MPO content in human and mouse plasma and atrial tissue.* We determined MPO levels by ELISA (human: Prognostix, Cleveland, USA; mouse: hycult biotechnology, The Netherlands).

*Detection of MPO, 3-chlorotyrosine and MMP-9.* We incubated sections of human right atrial appendages and mouse atrial tissue with an antibody to MPO (Calbiochem) and to chlorotyrosine (Hycult Biotechnology) antibodies. We assessed protein-bound 3-chlorotyrosine content of mouse and human hearts by stable isotope dilution liquid chromatography with on-line tandem mass spectrometry on a triple quadrupole mass spectrometer (LC-MS, ABI 3000 with redesigned source by Ionics, Concord, Canada) interfaced to an Aria LX Series HPLC (Cohesive Technologies Inc., Franklin, Massachusetts, USA)³⁴. In brief, prior to tissue homogenization and acid hydrolysis with methane sulfonic acid, we added synthetic [¹³C₆]-3-chlorotyrosine to samples as internal standard for quantification of 3-chlorotyrosine. Simultaneous monitoring for the potential formation of artifactual 3-chlorotyrosine (detected as [¹³C₉,¹⁵N] isotopomer) during sample preparation showed no detectable intrapreparative chlorination under the assay conditions employed. Pro-MMP-9 and MMP-9 was detected by western blot using a specific antibody (R&D systems).

*Histological assessment of fibrosis in mouse atria.* We applied picrosirius polarization method for quantitative evaluation of fibrosis³⁵. Quantitative analysis was performed by two independent pathologists blinded to individual treatment arms.

### Gelatin zymography. We performed zymography as previously

*Radiotelemetric blood pressure measurements.* We performed blood pressure measurements as previously³⁶. In brief, we inserted the tip of the telemetry device (TA11 PA-C10; Data Sciences International, St. Paul, USA) into the carotid lumen. After 10 days, data acquisition started for 1 minute every 5 minutes for 3 days. Thereafter, we started AngII, MPO or vehicle treatment. We recorded blood pressure and heart rate for the following week in MPO treated mice and two weeks in AngII treated animals, respectively.

*Rodent echocardiographic studies.* We determined maximal left atrial diameters in a modified parasternal long axis view using a Vevo 770 micro-imaging system equipped with a 30 MHz center frequency single element transducer.

*Human study outline.* Human studies, performed in accordance with the Declaration of Helsinki, were approved by the Board of Physicians Ethics Committee Hamburg and written informed consent was obtained. We included forty-two individuals with a newer generation dual chamber pacemaker in the study. Acute coronary syndrome within 1 month before study entry, decompensated heart failure, acute or chronic infections, malignancies, auto-immune disorders, renal failure, and severe valvular dysfunction were exclusion criteria. On presentation, we collected venous blood samples and carried out transthoracic echocardiography to assess ejection fraction (EF) according to Simpson's rule, functional status of the heart valves and left atrial diameter³⁷. We performed pacemaker interrogation obtaining intervention rate, time interval since the last interrogation and AF-burden at presentation and after the follow-up period. We defined the AF-burden as the percentage of time, during which the atrial rate of depolarization was above 200 beats per minute. The presence of AF was defined as an AF-burden greater than 0% according to the memory read-out of the pacemaker. Furthermore, we obtained right atrial appendages from 27 individuals undergoing elective coronary artery bypass surgery (ten individuals with persistent AF and 17 without AF). We determined plasma and atrial levels of MPO (Prognostix, Cleveland, USA) and plasma levels of elastase (IBL, Hamburg, Germany) by ELISA. Levels of atrial 3-chlorotyrosine were assessed by LC-MS.

*Statistical analysis.* The aim of the human study was to detect a minimal difference of 15% for MPO plasma levels (logarithmized because of an assumed non-normal distribution) between the two groups with a two-sided α of 0.05 and a power (1-β) of 0.80. Sample size calculation based on previous trials gave a formal sample size of *n* = 17. Categorical data are presented as frequencies and percentages and were compared by *x*² test. We tested continuous variables for normal distribution using the Kohnogorov-Smirnov test. Normally distributed variables are presented as meant standard deviation, for non-normally distributed data the median and interquartile range is given. To test for differences between groups we used Student's t-test for normally distributed data and Mann-Whitney U test for non-normally distributed data. In the case of multiple groups we employed one way ANOVA using Bonferroni post-hoc test or Kruskal-Wallis using Mann-Whitney U post hoc test, respectively. We assessed correlations between parameters using Spearman's correlation according to distribution of data. We considered a P-value < 0.05 as statistically significant. We carried out all statistical analyses with SPSS Version 15.0 (SPSS, Chicago, IL, USA).

The examples disclosed herein are supposed to illustrate and exemplify the invention, but not to limit its scope. Variations and modifications will be apparent to the skilled person. All references cited are fully incorporated herein by reference.

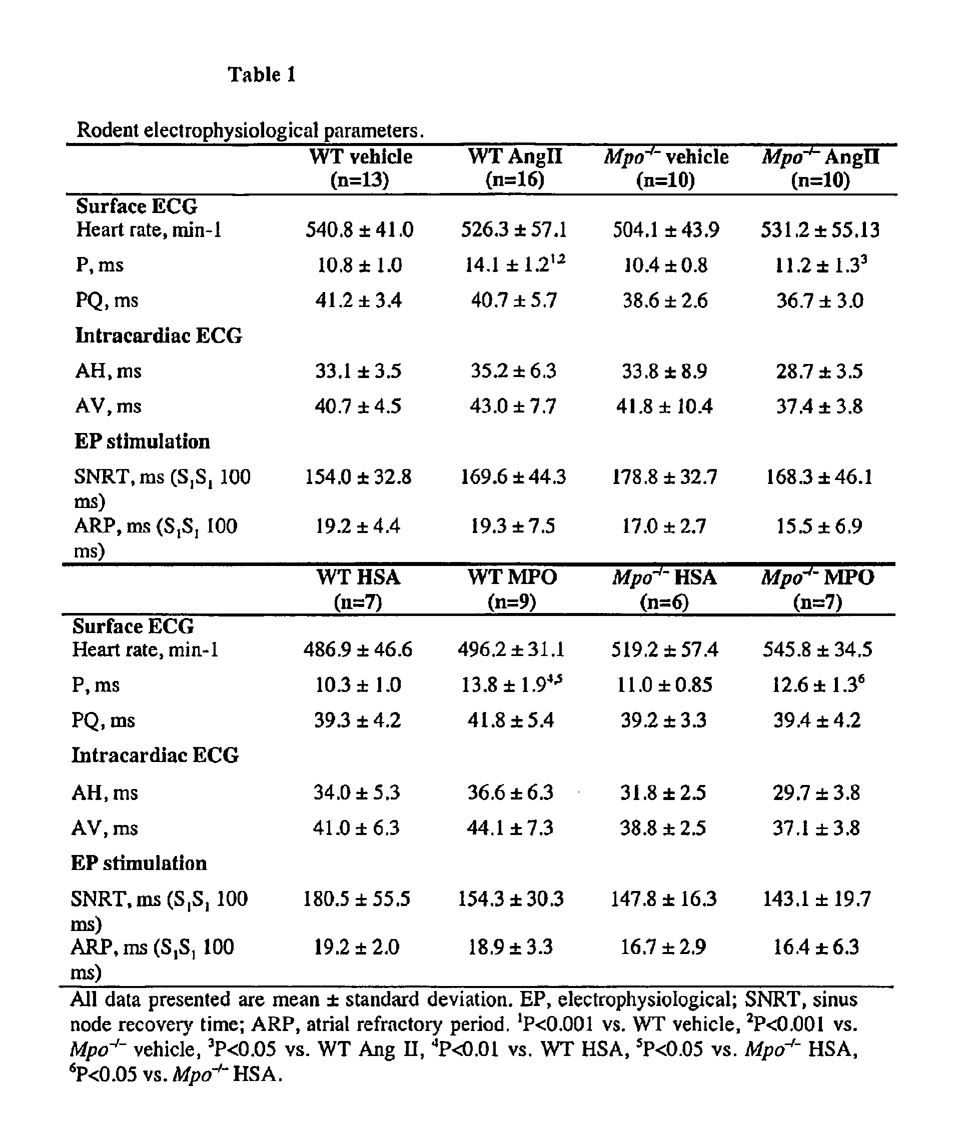

**Table 2**

| Patients' characteristics, analysis of plasma | | | |
|---|---|---|---|
| | **No AF** (n=18) | **AF** (n=24) | **p** |
| **Females** | 5(27.8) | 8(33.3) | 0.70 |
| **Age** | 68.5±8.3 | 70.1±6.8 | 0.50 |
| **BMI** | 24.2±7.0 | 24.0±5.4 | 0.94 |
| **Hypertension** | 15(83.3) | 21(87.5) | 0.40 |
| **Hypercholesterolemia** | 10(55.5) | 10(41.7) | 0.28 |
| **Diabetes mellitus** | 2(11.1) | 3(12.5) | 0.89 |
| **Smoking** | 0(0.0) | 0(0.0) | |
| **Family history** | 13(72.2) | 13(54.2) | 0.19 |
| **ASA** | 7(38.9) | 3(12.5) | 0.05 |
| **Oral Anticoagulation** | 6(33.3) | 19(79.2) | <0.05 |
| **Betablocker** | 6(33.3) | 12(50.0) | 0.28 |
| **ACE Inhibitor** | 12(66.6) | 10(41.7) | 0.11 |
| **AT1 Inhibitor** | 0(0.0) | 2(8.3) | 0.21 |
| **Statin** | 7(38.9) | 8(33.3) | 0.71 |
| **Hemoglobin, g/dL** | 14.4±0.9 | 14.0±1.3 | 0.22 |
| **Leukocyte count**, **1000/µl** | 5.8±1.1 | 5.8±1.4 | 0.99 |
| **Thrombocyte count 1000/µl** | 284.2±50.1 | 257.9±61.5 | 0.15 |
| **Creatinine, mg/dL** | 1.0±0.5 | 1.1±0.2 | 0.41 |
| **Total cholesterol**, **mg/dL** | 211.3±27.6 | 193.8±38.7 | 0.11 |
| **LDL cholesterol**, **mg/dL** | 110.7±19.9 | 102.9±30.1 | 0.35 |
| **HDL cholesterol**, **mg/dL** | 71.0±15.6 | 63.0±16.8 | 0.12 |
| **Ejection fraction**, **%** | 51.9±9.0 | 49.3±8.7 | 0.38 |
| **Mitral Regurgitation**, **I** | 9(50.0) | 12(50.0) | 0.43 |
| **II** | 2(11.1) | 6(25.0) | |
| **Left atrial diameter, mm** | 44.2±4.3 | 44.4±4.8 | 0.91 |
| **Pacemaker intervention rate, min⁻¹** | 57.5±15.7 | 57.5±13.6 | 1.00 |

Values are given as n(%) or mean ± standard deviation. ASA, acetylsalicylic acid; ACE, angiotensin-converting enzyme; AT1, angiotensin II type 1; BMI, body mass index; LDL, low density lipoprotein; HDL, high density lipoprotein.

**Table 3**

| Patients' characteristics, analysis of atrial tissue | | | |
|---|---|---|---|
| | No AF (n=17) | AF (n=10) | P |
| **Females** | 4(23.5) | 4(40) | 0.37 |
| **Age, years** | 68.6±13.8 | 71.0±6.5 | 0.60 |
| **Hypertension** | 10(58.8) | 7(70.0) | 0.69 |
| **Hypercholesterolemia** | 6(35.3) | 3(30.0) | 0.78 |
| **Diabetes mellitus** | 3(17.6) | 2(20.0) | 0.89 |
| **Smoking** | 5(29.4) | 1(10.0) | 0.36 |
| **ASA** | 14(82.4) | 1(10.0) | <0.05 |
| **Betablocker** | 12(70.6) | 4(40.0) | 0.22 |
| **ACE Inhibitor** | 10(58.8) | 6(60.0) | 0.95 |
| **Statin** | 13(76.5) | 5(50.0) | 0.22 |
| **Hemoglobin, g/dL** | 13.6±1.9 | 13.0±1.2 | 0.41 |
| **Leukocyte count**,**1000/ml** | 7.59±1.98 | 7.58±2.1 | 0.99 |
| **Creatinine, mg/dL** | 1.16±0.36 | 1.14±0.41 | 0.86 |

Values are given as n(%) or mean ± standard deviation. ASA, acetylsalicylic acid; ACE, angiotensin-converting enzyme.

### References

1. Issac, T. T., Dokainish, H. & Lakkis, N. M. Role of inflammation in initiation and perpetuation of atrial fibrillation: a systematic review of the published data. J Am Coll Cardiol 50, 2021-2028 (2007).
2. Kannel, W. B., Wolf, P. A., Benjamin, E. J. & Levy, D. Prevalence, incidence, prognosis, and predisposing conditions for atrial fibrillation: population-based estimates. Am J Cardiol 82, 2N-9N (1998).
3. Nattel, S. & Opie, L. H. Controversies in atrial fibrillation. Lancet 367, 262-272 (2006).
4. Kannel, W. B., Abbott, R. D., Savage, D. D. & McNamara, P. M. Epidemiologic features of chronic atrial fibrillation: the Framingham study. N Engl J Med 306, 1018-1022 (1982).
5. Aviles, R. J., et al. Inflammation as a risk factor for atrial fibrillation. Circulation 108, 3006-3010 (2003).
6. Sata, N., et al. C-reactive protein and atrial fibrillation. Is inflammation a consequence or a cause of atrial fibrillation? Jpn Heart J 45, 441-445 (2004).
7. Conway, D. S., Buggins, P., Hughes, E. & Lip, G. Y. Prognostic significance of raised plasma levels of interleukin-6 and C-reactive protein in atrial fibrillation. Am Heart J 148, 462-466 (2004).
8. Fontes, M. L., et al. Atrial fibrillation after cardiac surgery/cardiopulmonary bypass is associated with monocyte activation. Anesth Analg 101, 17-23, table of contents (2005).
9. Frustaci, A., et al. Histological substrate of atrial biopsies in patients with lone atrial fibrillation. Circulation 96, 1180-1184 (1997).
10. Nakamura, Y., et al. Tissue factor expression in atrial endothelia associated with nonvalvular atrial fibrillation: possible involvement in intracardiac thrombogenesis. Thromb Res 111, 137-142 (2003).
11. Chen, C. L., et al. Upregulation of matrix metalloproteinase-9 and tissue inhibitors of metalloproteinases in rapid atrial pacing-induced atrial fibrillation. J Mol Cell Cardiol 45, 742-753 (2008).
12. Fu, X., Kassim, S. Y., Parks, W. C. & Heinecke, J. W. Hypochlorous acid oxygenates the cysteine switch domain of pro-matrilysin (MMP-7). A mechanism for matrix metalloproteinase activation and atherosclerotic plaque rupture by myeloperoxidase. J Biol Chem 276, 41279-41287 (2001).
13. Fu, X., Kassim, S. Y., Parks, W. C. & Heinecke, J. W. Hypochlorous acid generated by myeloperoxidase modifies adjacent tryptophan and glycine residues in the catalytic domain of matrix metalloproteinase-7 (matrilysin): an oxidative mechanism for restraining proteolytic activity during inflammation. J Biol Chem 278, 28403-28409 (2003).
14. Baldus, S., et al. Endothelial transcytosis of myeloperoxidase confers specificity to vascular ECM proteins as targets of tyrosine nitration. J Clin Invest 108, 1759-1770 (2001).
15. Lin, C. S. & Pan, C. H. Regulatory mechanisms of atrial fibrotic remodeling in atrial fibrillation. Cell Mol Life Sci 65, 1489-1508 (2008).
16. Casaclang-Verzosa, G., Gersh, B. J. & Tsang, T. S. Structural and functional remodeling of the left atrium: clinical and therapeutic implications for atrial fibrillation. J Am Coll Cardiol 51, 1-11 (2008).
17. Burstein, B. & Nattel, S. Atrial fibrosis: mechanisms and clinical relevance in atrial fibrillation. J Am Coll Cardiol 51, 802-809 (2008).
18. Xiao, H. D., et al. Mice with cardiac-restricted angiotensin-converting enzyme (ACE) have atrial enlargement, cardiac arrhythmia, and sudden death. Am J Pathol 165, 1019-1032 (2004).
19. Roell, W., et al. Engraftment of connexin 43-expressing cells prevents post-infarct arrhythmia. Nature 450, 819-824 (2007).
20. Verheule, S., et al. Increased vulnerability to atrial fibrillation in transgenic mice with selective atrial fibrosis caused by overexpression of TGF-betal. Circ Res 94, 1458-1465 (2004).
21. Schrickel, J. W., et al. Enhanced heterogeneity of myocardial conduction and severe cardiac electrical instability in annexin A7-deficient mice. Cardiovasc Res 76, 257-268 (2007).
22. El Bekay, R., et al. Oxidative stress is a critical mediator of the angiotensin II signal in human neutrophils: involvement of mitogen-activated protein kinase, calcineurin, and the transcription factor NF-kappaB. Blood 102, 662-671 (2003).
23. Abu-Soud, H. M. & Hazen, S. L. Nitric Oxide Is a Physiological Substrate for Mammalian Peroxidases. Journal of Biological Chemistry 275, 37524-37532 (2000).
24. Eiserich, J. P., et al. Formation of nitric oxide-derived inflammatory oxidants by myeloperoxidase in neutrophils. Nature 391, 393-397 (1998).
25. Burstein, B., Qi, X. Y., Yeh, Y. H., Calderone, A. & Nattel, S. Atrial cardiomyocyte tachycardia alters cardiac fibroblast function: A novel consideration in atrial remodeling. Cardiovasc Res 76, 442-452 (2007).
26. Polyakova, V., Miyagawa, S., Szalay, Z., Risteli, J. & Kostin, S. Atrial extracellular matrix remodelling in patients with atrial fibrillation. J Cell Mol Med 12, 189-208 (2008).
27. Rossi, M. A. Pathologic fibrosis and connective tissue matrix in left ventricular hypertrophy due to chronic arterial hypertension in humans. J Hypertens 16, 1031-1041 (1998).
28. Hazen, S. L. & Heinecke, J. W. 3-Chlorotyrosine, a specific marker of myeloperoxidase-catalyzed oxidation, is markedly elevated in low density lipoprotein isolated from human atherosclerotic intima. J Clin Invest 99, 2075-2081 (1997).
29. Mihm, M. J., et al. Impaired myofibrillar energetics and oxidative injury during human atrial fibrillation. Circulation 104, 174-180 (2001).
30. Desrochers, P. E., Mookhtiar, K., Van Wart, H. E., Hasty, K. A. & Weiss, S. J. Proteolytic inactivation of alpha 1-proteinase inhibitor and alpha 1-antichymotrypsin by oxidatively activated human neutrophil metalloproteinases. J Biol Chem 267, 5005-5012 (1992).
31. Wang, Y., et al. Myeloperoxidase inactivates TIMP-1 by oxidizing its N-terminal cysteine residue: an oxidative mechanism for regulating proteolysis during inflammation. J Biol Chem 282, 31826-31834 (2007).
32. Schrickel, J. W., et al. Induction of atrial fibrillation in mice by rapid transesophageal atrial pacing. Basic Research in Cardiology 97, 452-460 (2002).
33. Schwoerer, A. P., et al. Mechanical unloading of the rat heart involves marked changes in the protein kinase-phosphatase balance. J Mol Cell Cardiol 45, 846-852 (2008).
34. Zheng, L., et al. Apolipoprotein AI is a selective target for myeloperoxidase-catalyzed oxidation and functional impairment in subjects with cardiovascular disease. Journal of Clinical Investigation 114, 529 (2004).
35. Lang, C., et al. Connective tissue growth factor: a crucial cytokine-mediating cardiac fibrosis in ongoing enterovirus myocarditis. J Mol Med 86, 49-60 (2008).
36. Butz, G. M. & Davisson, R. L. Long-term telemetric measurement of cardiovascular parameters in awake mice: a physiological genomics tool. Physiol Genomics 5, 89-97 (2001).
37. Cheitlin, M. D., et al. ACC/AHA/ASE 2003 guideline update for the clinical application of echocardiography: summary article: a report of the American College of Cardiology/American Heart Association Task Force on Practice Guidelines (ACC/AHA/ASE Committee to Update the 1997 Guidelines for the Clinical Application of Echocardiography). Circulation 108, 1146-1162 (2003).
38. Yea et al., Identification of a myeloperoxidase inhibitor from normal human serum._Clin Exp Immunol. 84(2): 347-352 (1991).
39. Kettle et al. Inhibition of myeloperoxidase by benzoic acid hydrazides,_Biochem J.; 308(Pt 2): 559-563 (1995).
40. Malle et al. Myeloperoxidase: a target for new drug development? Br J Pharmacol.152(6):838-54 (2007).
41. Jantschko et al., Exploitation of the unusual thermodynamic properties of human myeloperoxidase in inhibitor design. Biochem Pharmacol. 69(8):1149-57 (2005).
42. Pincemail et al., Human myeloperoxidase activity is inhibited in vitro by quercetin. Comparison with three related compounds. Cellular and Molecular Life Sciences 44: 450-453 (1988.)
43. US 2008096929
44. WO2006020498
45. US 2008009020
46. CA 2522413

## Claims

1. Inhibitor of expression and/or activity and/or atrial deposition of myeloperoxidase for use in the treatment of atrial fibrillation of a subject in need thereof.

2. The inhibitor of claim 1, wherein the inhibitor is an inhibitor of expression of myeloperoxidase selected from the group comprising siRNA and antisense RNA specifically inhibiting expression of myeloperoxidase.

3. The inhibitor of any of claims 1 or 2, wherein the inhibitor is an inhibitor of activity of myeloperoxidase selected from the group comprising quercitin, methimazole, rutin, rutin sulfate, troxerutin, a hydroxamic acid, a bencoic acid hydrazide or hydrazine, an indole or tryptamine derivative, flufenamic acid, diclofenac, niflumic acid, tenoxicam, piroxicam, melatonin, a derivative of 2,4-dihydro-[1,2,4]triazole-3-5 thione, a serum inhibitor of myeloperoxidase, and an antagonistic antibody to myeloperoxidase.

4. The inhibitor of any of claims 1 to 3, wherein the treatment is prophylactic and/or therapeutic treatment.

5. The inhibitor of any of claims 1 to 4, wherein the subject has been diagnosed with lone atrial fibrillation or with atrial fibrillation in combination with coronary artery disease and/or hypertension and/or heart failure and/or valvular disease, in particular mitral valve disease.

6. A method for diagnosing susceptibility to atrial fibrillation in a subject, comprising determining presence and/or activity of myeloperoxidase in a sample obtained from the subject.

7. A method for identifying a subject amenable to treatment with an inhibitor of claim 1, comprising determining presence and/or activity of myeloperoxidase in a sample obtained from the subject.

8. The method of any of claims 6 or 7, wherein the sample is selected from the group consisting of a blood sample, a plasma sample, a biopsy from the myocard and a specimen derived from right or left atrial appendages.

9. The method of any of claims 6 to 8, wherein the presence is determined by ELISA or Western blot.

10. The inhibitor of any of claims 1 to 5, wherein the subject has been determined to be amenable to treatment with an inhibitor of myeloperoxidase by the method of any of claims 7 to 9.

11. A method for identifying a compound for treatment of atrial fibrillation, comprising detecting activity or expression of myeloperoxidase in the presence and absence of a candidate substance under conditions suitable for myeloperoxidase activity, wherein the candidate substance is suitable for treatment of atrial fibrillation if it inhibits activity or expression of the myeloperoxidase.

12. Method of preparing a medicament for treatment of atrial fibrillation, comprising carrying out the method of claim 11 and formulating a compound identified as suitable for treatment of atrial fibrillation with a pharmacologically acceptable carrier and/or excipient.
